# EUROPEAN PATENT SPECIFICATION

(11) **EP 2 286 814 B1**
(45) Date of publication and mention of the grant of the patent: **16.10.2013**
(21) Application number: 09746617.1
(22) Date of filing: 13.05.2009
(51) Int. Cl.: A61K 31/473, A61K 9/70

(54) **TRANSDERMAL PREPARATION CONTAINING PALONOSETRON**
TRANSDERMALE ZUBEREITUNG MIT PALONOSETRON
PRÉPARATION TRANSDERMIQUE CONTENANT DE LA PALONOSÉTRONE

(30) Priority: 15.05.2008 US 71740 P
(43) Date of publication of application: 23.02.2011
(73) Proprietor: Hisamitsu Pharmaceutical Co., Inc., Tosu-shi, Saga 841-0017 (JP)
(72) Inventor: YASUKOCHI Takashi, Tsukuba-shi Ibaraki 305-0856 (JP); NAKA Yukihisa, Tsukuba-shi Ibaraki 305-0856 (JP); ENDO Tsuyoshi, Tsukuba-shi Ibaraki 305-0856 (JP); ADACHI Hirotoshi, Carlsbad, CA California 92008 (US)
(74) Representative: Grünecker, Kinkeldey, Stockmair & Schwanhäusser
(86) International application number: PCT/JP2009/058913
(87) International publication number: WO 2009/139411

(56) References cited:
- EP-A1- 1 366 762
- EP-A1- 1 625 845
- WO-A1-02/069942
- WO-A1-2006/124807
- WO-A1-2006/124807
- JP-A- H09 503 217
- JP-A- 2002 363 070
- JP-A- 2007 099 759
- US-A- 5 711 962

## Description

### Technical Field

The present invention relates to a percutaneous absorption preparation containing palonosetron.

### Background Art

Palonosetron hydrochloride is a serotonin subtype-3 (5-HT3) receptor antagonist, and it has high binding affinity for 5-HT3 receptor. The 5-HT3 receptor is localized in the autonomic nerves, enteric nervous system and sensory nerves, and has a function of promoting neural depolarization. The 5-HT3 receptor is also found in the chemoreceptor trigger zone (CTZ) of the medulla oblongata, and is known to be associated with vomiting reflex. The CTZ is outside of the blood brain barrier and can be directly stimulated from the blood, and at the floor of the fourth cerebral ventricle, the CTZ receives intracranial stimulation, vestibular stimulation, stimulation of such as metabolic disorder and emetic substances, and transmits the stimulation to the vomiting center of the medulla oblongata.

In 2003, palonosetron hydrochloride was approved in the U.S. as a drug for injection, and it has since been marketed. The target condition of palonosetron hydrochloride is the prevention or treatment of acute/prolonged CINV (Chemotherapy-Induced Nausea and Vomiting) accompanying initial or continuous treatment by moderate to advanced emetic cancer chemotherapy. The single dosage unit is 0.25 mg, administered 30 minutes before chemotherapy. Palonosetron hydrochloride can also be administered for prevention of PONV (Postoperative Nausea and Vomiting) up to 24 hours after surgery. It is also expected to have an effect on irritable bowel syndrome. Irritable bowel syndrome occurs as a result of abnormal motility and secretion in the large intestines and small intestines, producing symptoms of diarrhea or constipation.

Medical preparations containing palonosetron hydrochloride are used as injections, since they are aimed at patients with nausea, but have not been developed as practical percutaneous absorption preparation.

The following Patent Literatures exist as chief prior art relating to percutaneous absorption preparations containing 5-HT3 antagonists, and particularly palonosetron.

International Patent Publication No. WO2003/013482 discloses an invention relating to a percutaneous absorption preparation (tape, patch or other dressing) containing 5-HT3 antagonist. Ondansetron, granisetron, palonosetron and the like are listed as drugs. As preferred bases there are mentioned A-B-A type block copolymers obtained by crosslinking a hard segment (A) and a soft segment (B). It is described that precipitation of crystals is related to adhesion and drug content, and that components that inhibit drug crystallization in the adhesive may be added to the formulation. However, no suggestion is given regarding what types of components, specifically, are preferred to inhibit drug crystallization.

Japanese Unexamined Patent Publication HEI No. 10-167956 discloses a serotonin receptor antagonist-containing transdermal administration preparation. Japanese Unexamined Patent Publication HEI No. 10-167956 also discloses an antiemetic drug-administering device having a three-layer structure comprising a backing material layer, a drug storage layer and a pressure-sensitive adhesive layer (adhesive layer). Aliphatic alcohols are mentioned as preferred absorption accelerators. For the pressure-sensitive adhesive layer there are disclosed (meth)acrylic acid ester copolymers comprising hydroxyl group-containing monomers and carboxyl group-containing monomers. There is no particular suggestion of palonosetron or preventing crystal precipitation.

International Patent Publication No. WO1994/007468 discloses an invention relating to a two-phase matrix-type delayed release delivery system (sustained-release preparation) comprising a lipophilic polymer continuous phase and an aqueous phase, and it discloses a patch preparation with a support. The lipophilic polymer continuous phase of the matrix contains polyisobutylene as the pressure-sensitive adhesive, with a solvent base of polyacrylate or the like, and fatty alcohols are mentioned as components in the lipophilic polymer phase. Hydrophilic serotonin (5-HT3) receptor antagonists are mentioned as hydrophilic drugs, and specifically listed are ondansetron and granisetron. There is no particular suggestion of palonosetron or preventing crystal precipitation.

International Patent Publication No. WO2006/124807 discloses a composition for application to intact skin that has low skin irritation and that can be easily used for acute and delayed nausea and antiemesis, comprising a sustained-release 5-HT3 receptor antagonist, an absorption accelerator and an adhesive, as well as a treatment method using it. Granisetron and palonosetron are both listed as 5-HT3 receptor antagonist drugs. As absorption accelerators there are only disclosed typical esters of C 12-18 fatty acids and C1-6 alcohols, and it is stated that a large amount of percutaneous absorption accelerator tends to cause crystal precipitation of the 5-HT3 receptor antagonist, limiting its permeability.

Japanese Patent Public Inspection No. 2006-509739 discloses a transdermal delivery system for hydrophilic antiemetic agents, and a method for its use. Granisetron and ondansetron are listed as examples of antiemetic 5-HT3 receptor antagonists. The disclosed transdermal patch contains a penetration promoter and an adhesive layer. Specific examples of penetration promoters are not provided, and instead a mode without a penetration promoter is disclosed. The adhesive polymers disclosed include methacrylate polymers. However, there is no suggestion regarding fatty alcohols or preventing crystal precipitation.

Japanese Patent Public Inspection No. 2006-517944 discloses an invention relating to the use of palonosetron for treatment of postoperative nausea and vomiting, and it discloses injection and oral forms. While percutaneous administration is mentioned, the specific composition is not suggested. Moreover, there is also no suggestion regarding fatty alcohols or preventing crystal precipitation.

Japanese Patent Public Inspection No. 2006-508977 discloses an invention relating to the use of palonosetron for treatment of chemotherapy-induced vomiting, and it discloses injection and oral forms. While percutaneous administration is mentioned, the specific composition is not suggested. Moreover, there is also no suggestion regarding fatty alcohols or preventing crystal precipitation.

Japanese Unexamined Patent Publication No. 2005-170833 discloses an invention relating to a percutaneous absorption medical patch for serotonin (5-HT3) receptor antagonists. The adhesive bases disclosed are styrene-isoprene-styrene block copolymer and crosslinked acrylic adhesives. As serotonin receptor antagonists there are listed granisetron hydrochloride and ondansetron hydrochloride, with no specific mention of palonosetron, while it is disclosed that a propyleneglycol monofatty acid ester is included to promote absorption. Moreover, there is no suggestion regarding fatty alcohols or preventing crystal precipitation.

Japanese Patent Public Inspection No. 2002-536412 discloses a transdermal administration composition containing tropisetron, ondansetron, granisetron or the like as an antiemetic agent, with a hydrophilic organic solvent, a skin permeation accelerator and 15-80% water. Fatty alcohols are disclosed as skin permeation accelerators. Lauryl alcohol and oleyl alcohol are disclosed as fatty alcohols. There is no particular suggestion of palonosetron or preventing crystal precipitation. European Patent Applications EP 1 625 845 A1 and EP 1 366 762 A1 as well as US Patent Application No. US 5 711 962 A likewise mention a number of fatty alcohols as permeation enhancers, but do not disclose a composition containing palonosetron.

The following background art also exists as an ordinary percutaneous absorption system.

US Patent No. 6,586,000 discloses a method for increasing percutaneous absorption of a drug by a composition containing a hydroxide release agent such as an inorganic hydroxide, which is also a pH regulator, as an absorption accelerator. There is no specific mention of the drug palonosetron. It is disclosed that the pH of skin is preferably 8.5-13.0 at the site where the medical preparation is to be applied. Polysiloxanes, polyisobutylenes, polyacrylates and polystyrene-isoprene copolymers are disclosed as pressure-sensitive adhesives. Also disclosed as second absorption accelerators, to be preferably incorporated therein, are polyols such as polyethylene glycol, and sulfoxides such as DMSO. The invention is characterized by increasing cutaneous permeability and minimizing damage to skin.

Japanese Unexamined Patent Publication No. 2006-022057 discloses a percutaneous absorption preparation base containing 15-50% of a chain alcohol as an absorption accelerator. As chain alcohols there are mentioned isostearyl alcohol, hexyldecanol and octyldodecanol.

Japanese Unexamined Patent Publication No. 2002-363070 discloses a percutaneous absorption medical patch with inhibited crystal precipitation of antifungal drugs, by incorporation of a higher alcohol.

Japanese Patent Public Inspection HEI No. 9-503217 discloses a transdermal treatment system with an acrylate-based adhesive matrix containing estradiol and norethisterone as active components in a dissolved state, with octyldodecanol.

Japanese Patent Public Inspection No. 2002-518430 discloses a transdermal treatment system containing norethisterone acetate and estradiol, with a crystallization inhibitor and a polyacrylate pressure-sensitive adhesive. Several polymers are mentioned as crystallization inhibitors.

### Citation List

### Patent Literature

[Patent Literature 1] International Patent Publication No. WO2003/013482
[Patent Literature 2] Japanese Unexamined Patent Publication HEI No. 10-167956
[Patent Literature 3] International Patent Publication No. WO1994/007468
[Patent Literature 4] International Patent Publication No. WO2006/124807
[Patent Literature 5] Japanese Patent Public Inspection No. 2006-509739
[Patent Literature 6] Japanese Patent Public Inspection No. 2006-517944
[Patent Literature 7] Japanese Patent Public Inspection No. 2006-508977
[Patent Literature 8] Japanese Unexamined Patent Publication No. 2005-170833
[Patent Literature 9] Japanese Patent Public Inspection No. 2002-536412
[Patent Literature 10] US Patent No. 6,586,000
[Patent Literature 11] Japanese Unexamined Patent Publication No. 2006-022057
[Patent Literature 12] Japanese Unexamined Patent Publication No. 2002-363070
[Patent Literature 13] Japanese Patent Public Inspection HEI No. 9-503217
[Patent Literature 14] Japanese Patent Public Inspection No. 2002-518430
[Patent Literature 15] European Patent Application No. EP 1 625 845 A1
[Patent Literature 16] European Patent Application No. EP 1 366 762 A1
[Patent Literature 17] US Patent Application No. US 5 711 962 A

### Summary of Invention

### Technical Problem

As shown above, few concrete technical disclosures exist in the prior art regarding percutaneous absorption preparations containing palonosetron. Moreover, the disclosed art has been problematic in that it has not been possible to prepare the formulations using ordinary starting materials, which it has been necessary to add the drugs in large amounts, thus requiring provision of a storage layer of the drugs separate from the pressure-sensitive adhesive layer. These problems occur because of the high hydrophilicity of palonosetron, and because its solubility in ordinary oily percutaneous absorption systems is therefore low.

In addition, percutaneous absorption preparations containing palonosetron have presented practical problems because drug crystals precipitate easily during storage. As the drug crystals become precipitated, the percutaneous absorption of the drug is drastically reduced. Currently known techniques for preventing crystal precipitation have not been sufficient for percutaneous absorption preparations containing palonosetron, and therefore serious problems remain for the shelf life of such preparations.

It is an object of the invention to provide a percutaneous absorption preparation that has a simple laminated structure and an excellent shelf life, while maintaining excellent percutaneous absorption for palonosetron.

### Solution to Problem

The present inventors have conducted diligent research on percutaneous absorption preparations containing palonosetron, and have discovered as a result that a percutaneous absorption preparation with very high percutaneous absorption for palonosetron can be obtained, and that it can be produced as a tape preparation with a simple laminated structure. It was further discovered that addition of a fatty alcohol effectively prevents deposition of drug crystals with passage of time so that excellent percutaneous absorption of the drug can be maintained, and the present invention has been completed on this discovery.

Specifically, the invention provides a percutaneous absorption preparation comprising a support and a drug-containing pressure-sensitive adhesive layer formed on at least one side of the support, wherein the drug-containing pressure-sensitive adhesive layer comprises, as essential constituent components, an adhesive base having a styrene-based block copolymer as a base polymer, palonosetron or a pharmaceutically acceptable acid addition salt thereof, and at least one type of fatty alcohol selected from the group consisting of C6-12 straight-chain saturated alcohols, C 10-22 straight-chain unsaturated alcohols, C10-22 branched alcohols and C10-15 cyclic alcohols, and the constituent components other than palonosetron or its pharmaceutically acceptable acid addition salt are carboxyl group-free components.

In a percutaneous absorption preparation according to the invention, the constituent components other than palonosetron or its pharmaceutically acceptable acid addition salt contain no carboxyl groups, so that the solubility of the palonosetron in the drug-containing pressure-sensitive adhesive layer is increased, thus allowing the palonosetron to be held by a simple laminated structure without separate provision of a storage layer. Moreover, since the drug-containing pressure-sensitive adhesive layer contains the aforementioned specific fatty alcohol, crystal precipitation of palonosetron is inhibited and a percutaneous absorption preparation with an excellent shelf life can be obtained. Inhibiting precipitation of crystals can maintain high percutaneous absorption of palonosetron.

The fatty alcohol is preferably at least one selected from the group consisting of lauryl alcohol, octyldodecanol and isostearyl alcohol. This will yield a percutaneous absorption preparation with even more excellent effect of preventing precipitation of drug crystals.

The adhesive base is an adhesive base whose base polymer is a styrene-based block copolymer. This will yield a percutaneous absorption preparation with even more excellent percutaneous absorption for palonosetron.

### Advantageous Effects of Invention

According to the invention it is possible to obtain a percutaneous absorption preparation with a simple laminated structure which does not require a separate storage layer to hold palonosetron. The percutaneous absorption preparation has inhibited crystal precipitation of palonosetron and a lengthened shelf life, thus allowing higher percutaneous absorption of palonosetron to be maintained.

### Brief Description of Drawings

Fig. 1 is a graph showing the results of a permeation test for percutaneous absorption preparations using a hairless mouse skin.
Fig. 2 is a graph showing the results of a permeation test for percutaneous absorption preparations using a human skin.
Fig. 3 is a graph showing the results of a release test for percutaneous absorption preparations.

### Description of Embodiments

The percutaneous absorption preparation of the invention will now be described in detail. Throughout the present specification, "%" represents "mass%" unless otherwise specified.

The percutaneous absorption preparation of the invention is a preparation comprising a support, a drug-containing pressure-sensitive adhesive layer and a releasable protective layer, preferably laminated in that order. This type of percutaneous absorption preparation can be produced by simpler process. The drug-containing pressure-sensitive adhesive layer may be formed on both main sides of the support, or on only one side.

The drug-containing pressure-sensitive adhesive layer comprises as essential constituent components an adhesive base (pressure-sensitive adhesive base), palonosetron or a pharmaceutically acceptable acid addition salt thereof, with a fatty alcohol as mentioned above, and the constituent components other than palonosetron or its pharmaceutically acceptable acid addition salt are carboxyl group-free compounds.

Palonosetron or its pharmaceutically acceptable acid addition salt will now be explained first, as one of the constituent components of the drug-containing pressure-sensitive adhesive layer.

Palonosetron is a compound with the chemical name (3aS)-2-[(S)-1-azabicyclo[2.2.2]oct-3-yl]-2,3,3a,4,5,6-hexahydro-1-oxo-1H-1Hbenz[de]isoquinoline, and it is represented by the following chemical formula (1).

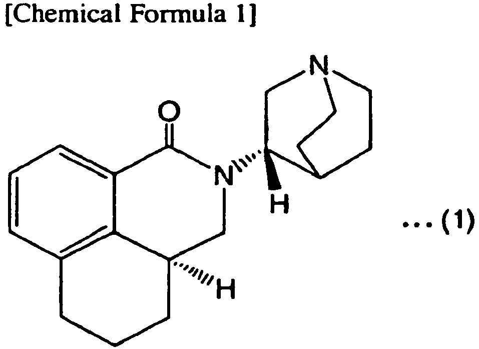

From the viewpoint of drug effect and side-effects, the palonosetron is preferably the (3aS)-2-[(S)-1-azabicyclo[2.2.2]oct-3-yl]-2,3,3a,4,5,6-hexahydro-1-oxo-1H-1Hbenz[de]isoquinoline isomer rather than a racemic mixture.

A pharmaceutically acceptable acid addition salt of palonosetron is a salt of an acid and palonosetron having the chemical structure shown above, and for example, it may be a hydrochloride, nitrate, phosphate, succinate, maleate, citrate, tartrate or gluconate of palonosetron. Palonosetron hydrochloride is preferred among these. Palonosetron hydrochloride is a drug approved by the FDA (Food and Drug Administration), having the chemical name (3aS)-2-[(S)-1-azabicyclo[2.2.2]oct-3-yl]-2,3,3a,4,5,6-hexahydro-1-oxo-1H-1Hbenz[de]isoquinoline hydrochloride, and its chemical formula is shown below as Chemical Formula (1a). Palonosetron hydrochloride has a molecular weight of 332.87, and the drug is a 5-HT3 receptor antagonist that is particularly useful in light of its effect of inhibiting delayed emesis.

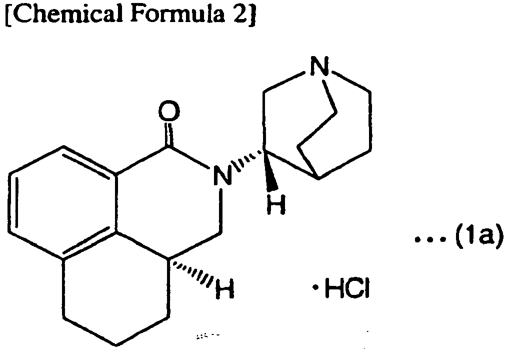

Palonosetron hydrochloride dissolves readily in water, while being slightly soluble in propylene glycol and poorly soluble in ethanol and 2-propanol, and it is a crystalline powder at room temperature.

Palonosetron and its pharmaceutically acceptable acid addition salt are included in the drug-containing pressure-sensitive adhesive layer in a range of preferably 0.3-10%, calculated as the free form (chemical formula (1)). As an antiemetic drug for sustained-release percutaneous absorption applied for a period of 1-7 days, palonosetron is preferably included in a range of 0.5-8%, calculated as the free form. The sustained-release percutaneous absorption preparation containing palonosetron is expected to have an effect of inhibiting delayed emesis, and has characteristics not found in other 5-HT3 receptor antagonists.

The percutaneous absorption preparation of the invention preferably has a skin permeation rate of 0.5-12 µg/cm²/hr for palonosetron or its pharmaceutically acceptable acid addition salt. The skin permeation rate is even more preferably 0.5-7 µg/cm²/hr, which will produce an effect of preventing and treating nausea and vomiting symptoms. If the skin permeation rate exceeds 12 µg/cm²/hr, side-effects such as headache and constipation may occur because the drug concentration in the blood is temporary increased excessively after administering preparation.

The adhesive base will now be explained, as one of the constituent components of the drug-containing pressure-sensitive adhesive layer.

The adhesive base itself is a base with an adhesive property in the temperature range at which the percutaneous absorption preparation is applied (preferably 0°C-50°C, more preferably 10°C-40°C and even more preferably 15°C-40°C). The adhesive base is an adhesive base whose base polymer is a styrene-based block copolymer.

Styrene-based block copolymers as base polymers generally do not exhibit an adhesive property by themselves. When a styrene-based block copolymer is used as the base polymer, therefore, a tackifier or softener is added to exhibit the adhesive property for use as the adhesive base.

As styrene-based block copolymers there may be mentioned styrene-isoprene-styrene block copolymer, styrene-butadiene-styrene block copolymer and styrene-ethylene/butylene-styrene block copolymer or the like, among which styrene-isoprene-styrene block copolymer is preferred.

As adhesive bases, there may be used mixtures of styrene-based block copolymers (e.g. styrene-isoprene-styrene block copolymer) and polyisobutylene, and mixtures of styrene-based block copolymers (e.g. styrene-isoprene-styrene block copolymer), polyisobutylene and (meth)acrylic acid ester copolymers.

The adhesive base described above must lack carboxyl groups. If an adhesive base with carboxyl groups is used, the percutaneous absorption of palonosetron will be reduced.

The adhesive base content is preferably in the range of 40-99% and more preferably in the range of 60-90%, based on the total amount of the drug-containing pressure-sensitive adhesive layer. If the content is less than 40%, the adhesion of the preparation onto skin will tend to be reduced.

The fatty alcohol will now be explained, as one of the constituent components of the drug-containing pressure-sensitive adhesive layer.

The fatty alcohol in the drug-containing pressure-sensitive adhesive layer is a liquid alcohol which can be a liquid state at 50°C or less with a function of preventing precipitation of palonosetron crystals with passage of time (crystal precipitation inhibitor). The fatty alcohol is at least one selected from the group consisting of C6-12 straight-chain saturated alcohols, C 10-22 straight-chain unsaturated alcohols, C 10-22 branched alcohols and C10-15 cyclic alcohols. Addition of a fatty alcohol can produce a preparation with inhibited precipitation of drug crystals with passage of time, and an excellent shelf life in terms of maintaining the high cutaneous permeability of the drug.

As C6-12 straight-chain saturated alcohols there are preferred C8-12 straight-chain saturated alcohols, among which octanol and lauryl alcohol are especially preferred. As C10-22 straight-chain unsaturated alcohols there are preferred C10-18 straight-chain unsaturated alcohols, among which oleyl alcohol and linoleyl alcohol are especially preferred, and linoleyl alcohol is most preferred. As C 10-22 branched alcohols there are preferred isostearyl alcohol, octyldodecanol and C10-15 branched terpene alcohols, from the viewpoint of absorption of palonosetron. Isostearyl alcohol is preferably used in the preparation of the invention in order to especially inhibit crystal precipitation of palonosetron. Even slight crystal precipitation during formulation of the preparation may result in extensive crystal precipitation during prolonged storage. By using isostearyl alcohol in the preparation of the invention it is possible to adjust easily the skin permeation rate of palonosetron to 2-7 µg/cm²/hr. As C10-15 branched terpene alcohols there are preferred branched monoterpene alcohols, with linalool and geraniol being more preferred. As C10-15 cyclic alcohols there are preferred C10-15 cyclic terpene alcohols. As C10-15 cyclic terpene alcohols there are preferred cyclic monoterpene alcohols, with menthol being more preferred.

The fatty alcohol content range is preferably 1-10%, and more preferably 2-5%, based on the total amount of the drug-containing pressure-sensitive adhesive layer. While the content of less than 1%, the effect of preventing precipitation of palonosetron crystals will tend to be reduced, while the content of greater than 10%, the adhesive property of the drug-containing pressure-sensitive adhesive layer will tend to be reduced. Particularly when isostearyl alcohol is used as a fatty alcohol, a content of isostearyl alcohol exceeding 10% will prevent uniform admixture with the adhesive base in the drug-containing pressure-sensitive adhesive layer, because of the high polarity of isostearyl alcohol, thus resulting in reduced cohesion and pressure-sensitive adhesion of the drug-containing pressure-sensitive adhesive layer.

When isostearyl alcohol is selected, the content of isostearyl alcohol is preferably 0.5-3 times the content of palonosetron. The content of less than 0.5 times will tend to result in no absorption-promoting effect or crystal precipitation-inhibiting effect. If the content exceeds 3 times, the adhesive property of the drug-containing pressure-sensitive adhesive layer will tend to be reduced.

The essential constituent components of the drug-containing pressure-sensitive adhesive layer have been explained above, and the following explanation concerns other components (additive components) that may be used in addition to the essential constituent components.

The drug-containing pressure-sensitive adhesive layer may further contain tackifiers, pH regulators, solubilizers, fillers, stabilizers, softeners or other additive components. If the adhesive base does not exhibit its adhesive property in the temperature range at which the percutaneous absorption preparation is to be applied, it will be necessary to add a tackifier. The additive components are appropriately added in a range of preferably not greater than 30%, more preferably not greater than 20% and even more preferably not greater than 10%, based on the total mass of the drug-containing pressure-sensitive adhesive layer.

As tackifiers there may be added tackifying resins such as terpene-based resins or petroleum-based resins, among which alicyclic saturated hydrocarbon resins are preferred. The softening point of the tackifier is preferably 60-160°C for excellent drug percutaneous absorption and adhesive properties.

A pH regulator, when used, may be an organic or inorganic acid, an organic or inorganic acid metal salt, a metal hydroxide, a metal oxide, or the like. Alkali metals and alkaline earth metals may be used as metals for organic or inorganic acid salts. Specifically, the pH regulator is preferably sodium lactate, sodium acetate, sodium hydroxide, or a combination of an acetic acid salt and acetic acid. The pH of the drug-containing pressure-sensitive adhesive layer is preferably in the range of 7.5-9.0. While the pH of the drug-containing pressure-sensitive adhesive layer is below 7.5, percutaneous absorption of the drug will tend to be reduced. While the pH of the drug-containing pressure-sensitive adhesive layer is higher than 9.0, risk of skin irritation will tend to increase. The pH of the drug-containing pressure-sensitive adhesive layer may be measured, for example, by placing a sample of the preparation with the releasable protective layer removed, having an actual area of 3 cm², in a 20 ml vial and adding 20 ml of purified water to the vial, agitating the vial for 3 days at 150 rpm and using an Orion 5Star pH Meter for measurement of the obtained liquid.

The solubilizer is preferably a polyol such as propylene glycol, dipropylene glycol or polyethylene glycol, or a sulfoxide such as dimethyl sulfoxide or decylmethyl sulfoxide. Addition of a solubilizer can increase the solubility of palonosetron in the drug-containing pressure-sensitive adhesive layer.

As softeners there may be mentioned nonpolar oils such as liquid paraffin, liquid polybutene, liquid isoprene, squalane and squalene or polar oils including vegetable oils (for example, hydrogenated castor oil, cottonseed oil, palm oil, coconut oil and the like), among which liquid paraffin is preferred from the standpoint of oxidation resistance and preventing precipitation of drug crystals.

The drug-containing pressure-sensitive adhesive layer is preferably "nonaqueous", i.e. containing essentially no water, and specifically the water content preferably does not exceed 10% based on the total amount of the drug-containing pressure-sensitive adhesive layer. A water content exceeding 10% will cause phase separation of the drug-containing pressure-sensitive adhesive layer, tending to produce undesirable results in terms of drug percutaneous absorption and adhesive property.

The thickness of the drug-containing pressure-sensitive adhesive layer is preferably 30-200 µm and more preferably 50-100 µm. A drug-containing pressure-sensitive adhesive layer thickness of less than 30 µm will tend to result in insufficient adhesion of the preparation onto skin, and a thickness of greater than 200 µm will cause cold flow and deformation of the drug-containing pressure-sensitive adhesive layer, thus tending to result in inconveniences during handling of the preparation.

The support is a sheet-like substance that physically supports the drug-containing pressure-sensitive adhesive layer and protects the drug-containing pressure-sensitive adhesive layer from the external environment. There are no special restrictions on the support, and it may be a known film, fabric, foamed sheet, microporous sheet, foil, or a laminated body of the foregoing, although it is preferably a film that is essentially non-permeable to the drug. The material of the support is preferably a polyester such as polyethylene terephthalate, polybutylene terephthalate or polyethylene naphthalate, a polyolefin such as polyethylene or polypropylene, or a metal such as aluminum. It is most preferred to use a polyester film in order to obtain a sustained-release preparation to be applied for several days, and from the viewpoint of flexibility and non-permeability to the drug.

The releasable protective layer is laminated to cover the drug-containing pressure-sensitive adhesive layer for protection from the external environment during storage, and it is released when the percutaneous absorption preparation is used. There are no special restrictions on the releasable protective layer, and it may be a known paper sheet, film, foil, or a laminated body of the foregoing, although it is preferably a film that is essentially non-permeable to the drug. The material of the releasable protective layer is preferably a polyester such as polyethylene terephthalate, polybutylene terephthalate or polyethylene naphthalate, a polyolefin such as polyethylene or polypropylene, a metal such as aluminum, or cellulose. The side of the releasable protective layer facing the drug-containing pressure-sensitive adhesive layer may be subjected to release treatment with silicone, Teflon (trademark) or the like to facilitate its release and removal, and treatment with silicone is particularly preferred to allow the release property to be stably maintained with the passage of time.

A percutaneous absorption preparation having such a construction is a more stable preparation exhibiting high percutaneous absorption for palonosetron and inhibit crystal precipitation during storage.

The percutaneous absorption preparation may be produced by an ordinary method such as, for example, evenly coating a releasable protective layer with a coating solution for the drug-containing pressure-sensitive adhesive layer, which comprises the adhesive base, palonosetron or its pharmaceutically acceptable acid addition salt, a fatty alcohol and other components dissolved or uniformly dispersed in a solvent, removing the solvent in the coated film to form a drug-containing pressure-sensitive adhesive layer, and then laminating a support thereover.

The solvent used may be any known solvent such as, for example, toluene, xylene, hexane, cyclohexane, ethyl acetate or propyl acetate. The preferred drying conditions for removal of the solvent in the coated film are 60-120°C for approximately 5-20 minutes.

### [Examples]

The present invention will now be explained in greater detail based on examples and comparative examples.

### (Working Example A)

### (Comparative Example 1)

In an organic solvent (toluene) there were dissolved 21.4 g (42.9mass%) of styrene-isoprene-styrene block copolymer (trade name: "Quintac3570C" by Nippon Zeon Corp.,), 4.3 g (8.6mass%) of liquid paraffin (trade name: "PRIMOL N382" by ExxonMobil), 21.4 g (42.9mass%) of an alicyclic saturated hydrocarbon resin with a softening point of 100°C (trade name: "ARKON P-100" by Arakawa Chemical Industries, Ltd.), 2.5 g of palonosetron hydrochloride (product of Hangzhou HETD Industry Co., Ltd.) (4.5mass% as free form) and 0.3 g (0.6mass%) of sodium hydroxide, to obtain a uniform coating solution. The solution was then coated onto a silicone treated 75 µm PET film as the releasable protective layer, and then the solvent was removed in an oven and a polyolefin/polyester laminated film was laminated as the support on the dried drug-containing pressure-sensitive adhesive layer, after which the laminate was cut to prescribed dimensions and the obtained preparation was sealed in a package.

### (Comparative Examples 2-10, Examples 1-3)

In an organic solvent (toluene) there were dissolved 20.8 g (41.5mass%) of styrene-isoprene-styrene block copolymer, 4.2 g (8.4mass%) of liquid paraffin, 20.8 g (41.5mass%) of an alicyclic saturated hydrocarbon resin with a softening point of 100°C, 2.5 g of palonosetron hydrochloride (4.5mass% as free form), 0.3 g (0.6mass%) of sodium hydroxide and 1.5 g (3.0mass%) of each crystal precipitation inhibitor shown in Table 1, to obtain a uniform coating solution. For Example 3, isostearyl alcohol (trade name: "RISONOL 18SP" by Kokyu Alcohol Kogyo Co., Ltd.) was used as the crystal precipitation inhibitor. The solution was then coated onto a silicone treated 75 µm PET film as the releasable protective layer, and then the solvent was removed in an oven and a polyolefin/polyester laminated film was laminated as the support on the dried drug-containing pressure-sensitive adhesive layer, after which the laminate was cut to prescribed dimensions and the obtained preparation was sealed in a package.

### (Comparative Example 11)

In an organic solvent (toluene) there were dissolved 20.3 g (40.6mass%) of styrene-isoprene-styrene block copolymer, 4.1 g (8.2mass%) of liquid paraffin, 20.3 g (40.6mass%) of an alicyclic saturated hydrocarbon resin with a softening point of 100°C, 2.5 g of palonosetron hydrochloride (4.5mass% as free form), 0.3 g (0.6mass%) of sodium hydroxide and 2.5 g (5.0mass%) of propylene glycol as a crystal precipitation inhibitor, to obtain a uniform coating solution. The solution was then coated onto a silicone treated 75 µm PET film as the releasable protective layer, and then the solvent was removed in an oven and a polyolefin/polyester laminated film was laminated as the support on the dried drug-containing pressure-sensitive adhesive layer, after which the laminate was cut to prescribed dimensions and the obtained preparation was sealed in a package.

### (Comparative Example 12)

In an organic solvent (toluene) there were dissolved 19.9 g (39.7mass%) of styrene-isoprene-styrene block copolymer, 4.0 g (8.0mass%) of liquid paraffin, 19.9 g (39.7mass%) of an alicyclic saturated hydrocarbon resin with a softening point of 100°C, 2.5 g of palonosetron hydrochloride (4.5mass% as free form), 0.3 g (0.6mass%) of sodium hydroxide and 3.5 g (7.0mass%) of propylene glycol as a crystal precipitation inhibitor, to obtain a uniform coating solution. The solution was then coated onto a silicone treated 75 µm PET film as the releasable protective layer, and then the solvent was removed in an oven and a polyolefin/polyester laminated film was laminated as the support on the dried drug-containing pressure-sensitive adhesive layer, after which the laminate was cut to prescribed dimensions and the obtained preparation was sealed in a package.

**[Table 1]**

| Preparation | Crystal precipitation inhibitor | Content (%) |
|---|---|---|
| Comp. Ex. 1 | None | 0 |
| Comp. Ex. 2 | Propylene glycol | 3 |
| Comp. Ex. 3 | Dimethyl isosorbide | 3 |
| Comp. Ex. 4 | Isopropyl palmitate | 3 |
| Comp. Ex. 5 | Crospovidone | 3 |
| Comp. Ex. 6 | Polyvinyl pyrrolidone | 3 |
| Comp. Ex. 7 | Kaolin | 3 |
| Comp. Ex. 8 | Bentonite | 3 |
| Example I | Lauryl alcohol | 3 |
| Comp. Ex. 9 | Myristyl alcohol | 3 |
| Example 2 | Octyldodecanol | 3 |
| Comp. Ex. 10 | Stearyl alcohol | 3 |
| Example 3 | Isostearyl alcohol | 3 |
| Comp. Ex. 11 | Propylene glycol | 5 |
| Comp. Ex. 12 | Propylene glycol | 7 |

### [Evaluation of crystal precipitation]

Each of the above-mentioned preparations was stored for a prescribed period of time in a stability test chamber at 5°C or 25°C and then removed, and the amount of drug crystals produced in the drug-containing pressure-sensitive adhesive layer was visually observed and evaluated on the following scale. The evaluation results are shown in Table 2.
A: No crystal precipitation (Very good).
B: Slight crystal precipitation (Good).
C: Small amount of crystal precipitation (Fair).
D: Large crystal precipitation (Poor).
E: Very large crystal precipitation (Very poor).
-: Not evaluated.

**[Table 2]**

| Storage temperature | 5°C | 5°C | 25°C | 25°C | 25°C |
|---|---|---|---|---|---|
| Storage period | 1 week | 2 week | 1 week | 2 week | 1 month |
| Comp. Ex. 1 | D | E | C | D | E |
| Comp. Ex. 2 | A | B | A | A | A |
| Comp. Ex. 3 | D | E | B | D | E |
| Comp. Ex. 4 | D | E | C | E | E |
| Comp. Ex. 5 | D | E | B | D | E |
| Comp. Ex. 6 | D | D | B | D | E |
| Comp. Ex. 7 | D | D | B | D | E |
| Comp. Ex. 8 | D | E | D | E | E |
| Example 1 | - | B | - | - | - |
| Comp. Ex. 9 | - | E | - | - | - |
| Example 2 | - | B | - | - | - |
| Comp. Ex. 10 | - | E | - | - | - |
| Example 3 | - | A | - | - | - |
| Comp. Ex. 11 | - | A | - | - | - |
| Comp. Ex. 12 | - | A | - | - | - |

### [Physical property evaluation and peel test]

After obtaining each preparation, it was immediately subjected to the following physical property evaluation and peel test.

### (Physical property evaluation)

Each preparation was visually observed for separation of the components or color abnormalities. Also, the releasable protective layer was removed and the drug-containing pressure-sensitive adhesive layer was touched with a finger and the cohesion and adhesive property of the drug-containing pressure-sensitive adhesive layer were evaluated on the following scale. The evaluation results are shown in Table 3.
A: No separation (bleeding) of components and no coloring, excellent cohesion and pressure-sensitive adhesion of drug-containing pressure-sensitive adhesive layer (Good).
B: Inferior cohesion and pressure-sensitive adhesion of drug-containing pressure-sensitive adhesive layer (Poor).
C: Bleeding or other abnormal outer appearance (Very poor).
-: Not evaluated.

### (Peel test method)

### Preparation of samples:

Each preparation was cut to a rectangular shape with a width of 10 mm and a length of 50 mm, and after releasing the releasable protective layer, it was attached to a Bakelite test board for use as a sample.

### Test method:

Each sample was mounted in an Instron tensile tester (trade name: "TENSILON RTM-100" by Orientech Co., Ltd.) and peeled at 180° at a fixed speed of 300 mm/min, and the peel strength was measured after the start of peeling at 5 points at 5 mm intervals from an initial position of 5 mm, with the average value being recorded as the peel value (unit: gf / 10mm).

**[Table 3]**

| Preparation | Physical properties | Peel test |
|---|---|---|
| Comp. Ex. 1 | - | 347 |
| Comp. Ex. 2 | B | 490 |
| Comp. Ex. 3 | - | 594 |
| Comp. Ex. 4 | - | - |
| Comp. Ex. 5 | - | 540 |
| Comp. Ex. 6 | - | 545 |
| Comp. Ex. 7 | - | 572 |
| Comp. Ex. 8 | - | - |
| Example 1 | A | 432 |
| Comp. Ex. 9 | A | 481 |
| Example 2 | A | 469 |
| Comp. Ex. 10 | B | - |
| Example 3 | A | 486 |
| Comp. Ex. 11 | C | 382 |
| Comp. Ex. 12 | C | 259 |

### [Permeation test using hairless mouse skin]

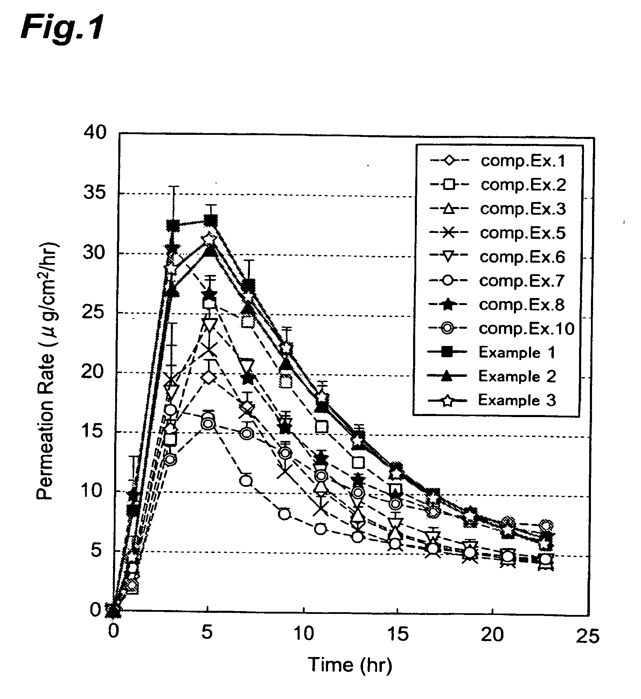

A 5 cm² round test preparation was attached to the horny layer side of subcutaneous fat-removed skin that had been extracted from a hairless mouse (female, 7 weeks old), and it was inserted in a flow-through diffusion cell with hot water at 32°C circulating around the outer periphery, with the dermis side on the receptor reservoir side. Phosphate-buffered saline (pH 7.4) was used as the medium in the receptor reservoir, and the solvent was sampled every 12 hrs up to a prescribed time, at a flow rate of about 5 ml/hr. The flow rate of the obtained solvent was precisely measured, the drug concentration was determined by high-performance liquid chromatography (HPLC), the permeation rate per hour at each sampling point was calculated, and the skin permeation rate of the drug was calculated according to the following formula. The measurement results are shown in Fig. 1. Skin permeation rate (µg/cm²/hr) = {Sample concentration (µg/ml) × flow rate (ml)}/application area of preparation (cm²)

As a result of the evaluation, Examples 1-3, which contained branched or unsaturated fatty alcohols used as crystal precipitation inhibitors according to the invention, exhibited high cutaneous permeability of the drug and inhibit drug crystal precipitation during storage, and were therefore excellent percutaneous absorption preparations.

In Comparative Examples 2, 11 and 12, an effect of inhibiting drug crystal precipitation was obtained with propylene glycol, which can also be used as a solubilizer. However, the effect was greatly inferior compared to the fatty alcohols such as isostearyl alcohol.

### (Working Example B)

### (Examples 4-7, Comparative Example 13-16)

In an organic solvent (toluene) there were dissolved styrene-isoprene-styrene block copolymer (trade name: "Quintac3570C" by Nippon Zeon Corp.,), liquid paraffin (trade name: "PRIMOL N382" by ExxonMobil), an alicyclic saturated hydrocarbon resin with a softening point of 100°C (trade name: "ARKON P-100" by Arakawa Chemical Industries, Ltd.), palonosetron hydrochloride (product of Hangzhou HETD Industry Co., Ltd.), isostearyl alcohol (trade name: "RISONOL 18SP" by Kokyu Alcohol Kogyo Co., Ltd.), polyisobutylene (trade name: "Oppanol" by BASF Corp.) and sodium hydroxide with contents shown in Table 4 and 5, to obtain a uniform coating solution. The solution was then coated onto a silicone treated 75 µm PET film as the releasable protective layer, and then the solvent was removed in an oven and a polyolefin/polyester laminated film was laminated as the support on the dried drug-containing pressure-sensitive adhesive layer, after which the laminate was cut to prescribed dimensions and the obtained preparation was sealed in a package.

**[Table 4]**

| Preparation | | Example 4 | Example 5 | Example 6 | Example 7 |
|---|---|---|---|---|---|
| Content (mass%) | Palonosetron hydrochloride (as free form) | 2 | 3 | 4 | 5 |
| | Isostearyl alcohol | 3 | 3 | 3 | 3 |
| | Styrene-isoprene-styrene block copolymer | 16.42 | 16.22 | 16.02 | 15.84 |
| | Alicyclic saturated hydrocarbon resin | 43.96 | 43.44 | 42.92 | 42.40 |
| | Liquid paraffin | 27.35 | 27.03 | 26.71 | 26.38 |
| | polyisobutylene | 7.03 | 6.95 | 6.87 | 6.78 |
| | sodium hydroxide | 0.24 | 0.36 | 0.48 | 0.60 |

**[Table 5]**

| Preparation | | Comp. Ex. 13 | Comp. Ex. 14 | Comp. Ex. 15 | Comp. Ex. 16 |
|---|---|---|---|---|---|
| Content (mass%) | Palonosetron hydrochloride (as free form) | 2 | 3 | 4 | 5 |
| | Isostearyl alcohol | 0 | 0 | 0 | 0 |
| | Styrene-isoprene-styrene block copolymer | 16.93 | 16.74 | 16.54 | 16.35 |
| | Alicyclic saturated hydrocarbon resin | 45.35 | 44.83 | 44.32 | 43.79 |
| | Liquid paraffin | 28.22 | 27.9 | 27.57 | 27.25 |
| | polyisobutylene | 7.26 | 7.17 | 7.09 | 7.01 |
| | sodium hydroxide | 0.24 | 0.36 | 0.48 | 0.60 |

### [Evaluation of crystal precipitation]

Each of the above-mentioned preparations was stored for a prescribed period of time in a stability test chamber at each temperature (5°C, 25°C, 40°C and 60°C) and then removed, and the drug crystals produced in the drug-containing pressure-sensitive adhesive layer was observed visually and with optical microscope, and elapsed time until drug crystals were precipitated was evaluated. The evaluation results are shown in Table 6.

**[Table 6]**

| Preparation | Storage temperature | | | |
|---|---|---|---|---|
| | 5°C | 25°C | 40°C | 60°C |
| Example 4 | None | None | None | None |
| Comp. Ex. 13 | None | None | 14 days | None |
| Example 5 | None | None | 14 days | None |
| Comp. Ex. 14 | 25 days | 14 days | 4 days | 7 days |
| Example 6 | None | None | 7 days | 14 days |
| Comp. Ex. 15 | 1 day | 3 days | 1 day | 1 day |
| Example 7 | 19 days | 25 days | 4 days | 9 days |
| Comp. Ex. 16 | 1 day | 3 days | 1 day | 1 day |

### (Working Example C)

### [Evaluation of adhesive base function]

Palonosetron acid addition salts were evaluated to determine the effect of the functional group of the adhesive base on cutaneous permeability of palonosetron in percutaneous absorption preparations. The methods for obtaining the preparations and conducting a permeation test using a human skin are described below.

### [Percutaneous absorption preparations]

As adhesive bases there were used the acrylic compound "Duro-Tak 87-4287" having hydroxyl groups but no carboxyl groups, the acrylic compound "Duro-Tak 87-4098" having no carboxyl or hydroxyl groups, the compound "Duro-Tak 87-2196" having carboxyl groups but no hydroxyl groups and the compound "Duro-Tak 87-2074" having hydroxyl and carboxyl groups (procured from Henkel Corp.), polyisobutylene procured from ExxonMobil, and the silicone compound "PSA 7-4101" procured from Dow Coming, Inc. Percutaneous absorption preparations were obtained by combining each of these with 1.0% of palonosetron hydrochloride as the free form and 10% propylene glycol as a solubilizer based on the total amount of drug-containing pressure-sensitive adhesive layer, as shown in Table 7.

The propylene glycol was added to the palonosetron hydrochloride and the mixture was stirred for 2 hours. The remaining reagent was then added and stirred therewith to uniformity to obtain a coating solution. Each coating solution was then coated onto a silicone surface-treated 75 µm polyester film as the releasable protective layer, and then the solvent was removed in an oven and a polyolefin/polyester laminated film was laminated as the support on the dried drug-containing pressure-sensitive adhesive layer, after which the laminate was cut to a prescribed size and the obtained preparation was sealed in a package.

**[Table 7]**

| Preparation | Acid addition salt of Palonosetron | Adhesive base | Solubilizer |
|---|---|---|---|
| Preparation 1 | Palonosetron hydrochloride | Duro-Tak 87-4287 | Propylene glycol |
| Preparation 2 | Palonosetron hydrochloride | Duro-Tak 87-4098 | Propylene glycol |
| Preparation 3 | Palonosetron hydrochloride | Polyisobutylene | Propylene glycol |
| Preparation 4 | Palonosetron hydrochloride | Si licone | Propylene glycol |
| Preparation 5 | Palonosetron hydrochloride | Duro-Tak 87-2196 | Propylene glycol |
| Preparation 6 | Palonosetron hydrochloride | Duro-Tak 87-2074 | Propylene glycol |

### [In vitro human skin permeation test]

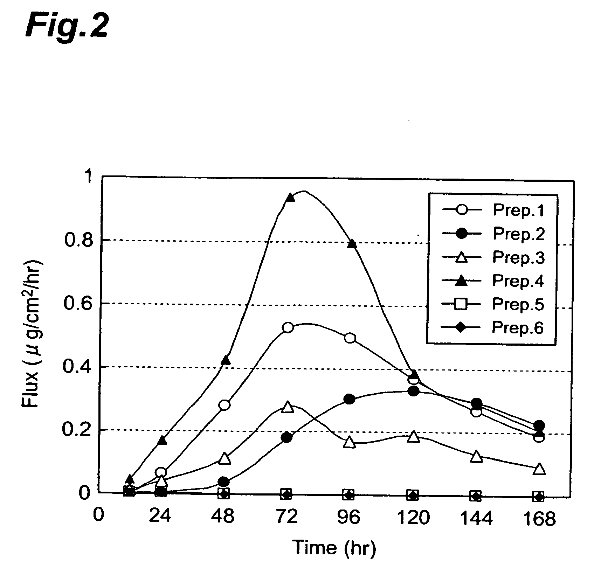

A test preparation was attached to the horny layer side of skin excised from a human cadaver, and it was inserted in a flow-through diffusion cell with hot water at 32°C circulating around the outer periphery, with the dermis side on the receptor reservoir side. A phosphoric acid/physiological saline buffering solution at pH 7.4 was used as the medium in the receptor reservoir, and the solvent was sampled every 12 hrs at a fixed flow rate, up to 168 hours. The flow rate of the obtained solvent was precisely measured, the drug concentration was determined by HPLC, the permeation rate per hour at each sampling point was calculated, and the skin permeation rate of the drug was calculated according to the same formula used for the skin permeation test using hairless mouse. The measurement results are shown in Fig. 2.

As shown in Fig. 2, Preparations 5 and 6 which employed adhesive bases with carboxyl groups had very low drug cutaneous permeabilities of less than 0.1 µg/cm²/hr, while Preparations 1-4 which employed adhesive bases without carboxyl groups exhibited higher drug cutaneous permeabilities of 0.1 µg/cm²/hr or greater.

[Evaluation of controlled release of adhesive bases] Percutaneous absorption preparations were obtained in the same manner as Working Example A. The adhesive bases used were the acrylic compound "Duro-Tak 87-4287" having hydroxyl groups but no carboxyl groups and the acrylic compound "Duro-Tak 87-4098" having no carboxyl or hydroxyl groups, the compound, both procured from Henkel Corp.. Percutaneous absorption preparations were obtained by combining these with 1.0% of palonosetron hydrochloride as the free form and 6% propylene glycol, based on the total amount of drug-containing pressure-sensitive adhesive layer, as shown in Table 8.

**[Table 8]**

| Preparation | Acid addition salt of Palonosetron | Adhesive base | Solubilizer |
|---|---|---|---|
| Preparation 7 | Palonosetron hydrochloride | Duro-Tak 87-4287 | Propylene glycol |
| Preparation 8 | Palonosetron hydrochloride | Duro-Tak 87-4098 | Propylene glycol |
| Preparation 9 | Palonosetron hydrochloride | 1:1 solid mass mixture of Duro-Tak 97-4287 and Duro-Tak 874098 | Propylene glycol |

### [Release test]

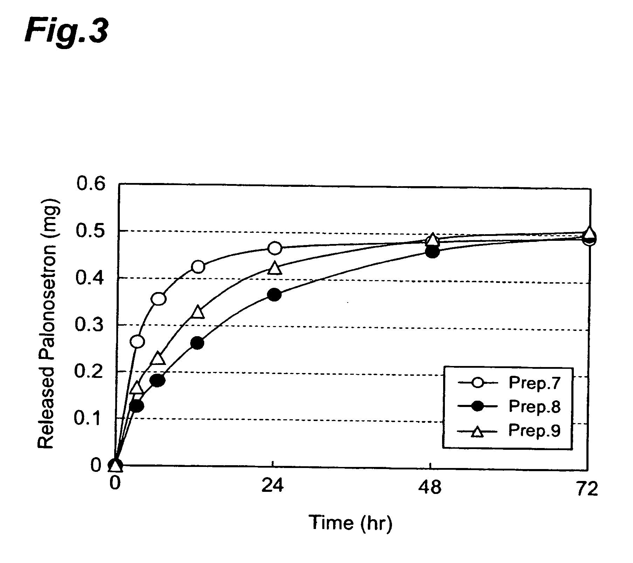

A release test was carried out based on the water release test using a rotary cylinder, described in the U.S. Pharmacopeia Release Test Method. The preparation was punched out to 5 cm², and the support side of the preparation was affixed to the barrel of the rotary cylinder with a silicone adhesive. The releasable protective layer was removed for the release test. A 900 ml portion of phosphate buffer (10 mM, pH 7.4, 32°C) was set in an elution tester and the rotary cylinder was dipped in the phosphate buffer and agitated at a rotational speed of 100 rpm. A 2 ml portion of solution was taken after a prescribed period, the drug concentration in the sampling solution was measured by HPLC, and the water release at each time point was calculated. The cumulative release for palonosetron was measured in this manner. The measurement results are shown in Fig. 3.

As shown in Fig. 3, Preparation 7 exhibited a rapid-release property while Preparation 8 exhibited a sustained-release property. Preparation 9, which had a mixed composition comprising Preparations 7 and 8 in a solid mass ratio of 1:1, had moderate rapid-release and sustained-release properties. It was thus demonstrated that desired drug release properties can be obtained by varying the mixing proportion of these adhesive bases.

### Industrial Applicability

The palonosetron percutaneous absorption preparation of the invention has a simple laminated structure while also exhibiting excellent percutaneous absorption and a long shelf life, and it is therefore very useful in the medical field, and especially for the prevention and treatment of cancer chemotherapy-induced vomiting.

## Claims

1. A percutaneous absorption preparation comprising a support and a drug-containing pressure-sensitive adhesive layer formed on at least one side of the support, wherein the drug-containing pressure-sensitive adhesive layer comprises as essential constituent components
an adhesive base,
palonosetron or a pharmaceutically acceptable acid addition salt thereof, and
at least one type of fatty alcohol selected from the group consisting of C6-12 straight-chain saturated alcohols, C10-22 straight-chain unsaturated alcohols, C10-22 branched alcohols and C10-15 cyclic alcohols, and
the constituent components other than palonosetron or its pharmaceutically acceptable acid addition salt are carboxyl group-free components,
wherein the base polymer of the adhesive base is a styrene-based block copolymer.

2. The percutaneous absorption preparation according to claim 1, wherein the fatty alcohol is at least one selected from the group consisting of lauryl alcohol, octyldodecanol and isostearyl alcohol.

## Patentansprüche

1. Perkutane Absorptionszubereitung, umfassend einen Träger und eine arzneimittelhaltige Haftklebstoffschicht, die auf wenigstens einer Seite des Trägers gebildet ist, wobei die arzneimittelhaltige Haftklebstoffschicht als wesentliche konstituierende Komponenten einen Klebstoffgrundstoff,
Palonosetron oder ein pharmazeutisch verträgliches Säureadditionssalz davon, und
wenigstens eine Art von Fettalkohol, ausgewählt aus der Gruppe bestehend aus geradkettigen gesättigten C6-12-Alkoholen, geradkettigen ungesättigten C10-22-Alkoholen, verzweigten C10-22-Alkoholen und cyclischen C10-15-Alkoholen
umfasst, und
die konstituierenden Komponenten, außer Palonosetron oder seinem pharmazeutisch verträglichen Säureadditionssalz, carboxylgruppen-freie Komponenten sind,
wobei das Grundpolymer des Klebstoffgrundstoffs ein Block-Copolymer auf Styrolbasis ist.

2. Perkutane Absorptionszubereitung nach Anspruch 1, wobei der Fettalkohol wenigstens ein Alkohol, ausgewählt aus der Gruppe bestehend aus Laurylalkohol, Octyldodecanol und Isostearylalkohol, ist.

## Revendications

1. Préparation pour l'absorption percutanée comprenant un support et une couche adhésive sensible à la pression contenant un médicament formée sur au moins un côté du support, dans laquelle la couche adhésive sensible à la pression contenant un médicament comprend comme composants constitutifs essentiels
une base adhésive,
de la palonosétrone ou un sel d'addition d'acide pharmaceutiquement acceptable de celle-ci, et
au moins un type d'alcool gras sélectionné parmi le groupe constitué des alcools saturés à chaîne linéaire en C₆ à C₁₂, des alcools insaturés à chaîne linéaire en C₁₀ à C₂₂, des alcools ramifiés en C₁₀ à C₂₂ et des alcools cycliques en C₁₀ à C₁₅, et
les composants constitutifs autres que la palonosétrone ou son sel d'addition d'acide pharmaceutiquement acceptable sont des composants exempts de groupe carboxyle,
dans laquelle le polymère de base de la base adhésive est un copolymère séquencé à base de styrène.

2. Préparation pour l'absorption percutanée selon la revendication 1, dans laquelle l'alcool gras est au moins l'un sélectionné parmi le groupe constitué de l'alcool de lauryle, de l'octyldodécanol et de l'alcool d'isostéaryle.
